# EUROPEAN PATENT SPECIFICATION

(11) **EP 0 595 849 B1**
(45) Date of publication and mention of the grant of the patent: **25.11.1998**
(21) Application number: 92914481.4
(22) Date of filing: 08.07.1992
(51) Int. Cl.: A61B 17/22

(54) **USE OF COMPOSITE PIEZOELECTRIC TRANSDUCER FOR ULTRASONIC THERAPY APPARATUS**
VERWENDUNG VON PIEZOELEKTRISCHEM KOMPOSITWANDLER FÜR ULTRASONISCHE THERAPIEVORRICHTUNG
UTILISATION D'UN TRANSDUCTEUR PIEZOELECTRIQUE DANS LA FABRICATION D'UN APPAREIL THERAPEUTIQUE ULTRASONIQUE

(30) Priority: 19.07.1991 FR 9109197
(43) Date of publication of application: 11.05.1994
(73) Proprietor: TECHNOMED MEDICAL SYSTEMS, 69500 Bron (FR); INSTITUT NATIONAL DE LA SANTE ET DE LA RECHERCHE MEDICALE (INSERM), 75654 Paris Cédex 13 (FR)
(72) Inventor: CHAPELON, Jean-Yves, F-69100 Villeurbanne (FR); CATHIGNOL, Dominique, F-69740 Genas (FR); BLANC, Emmanuel, F-69230 S.-Genis-Laval (FR)
(74) Representative: Hirsch, Marc-Roger
(86) International application number: EP9201534
(87) International publication number: WO9301752

(56) References cited:
- EP-A- 137 529
- EP-A- 0 326 701
- EP-A- 0 419 872
- GB-A- 2 126 901
- US-A- 4 721 106
- US-A- 4 858 597
- IEEE 1989 ULTRASONICS SYMPOSIUM vol. 2, 3 October 1989, MONTREAL, CANADA pages 755 - 766 SMITH, WALLACE ARDEN 'The role of piezocomposites in ultrasonic transducers' cited in the application
- PIEZOLITH 2300 September 1988, RICHARD WOLF GMBH

## Description

The invention relates to the use of at least one composite piezoelectric transducer for the manufacture of a therapy apparatus for applying destructive treatment in a body zone, in particular to tissues of a living being; it also relates to an acoustic or ultrasonic therapy apparatus for applying destructive treatment in a body zone, in particular to tissues, of a living being, the apparatus comprising at least one piezoelectric transducer, and electrical feed means including pulse generators for causing said transducer to provide a sufficient dosis of acoustic energy to perform said destructive treatment in said body zone.

### BACKGROUND OF THE INVENTION

The existence of composite piezoelectric transducers is quite recent. Composite piezoelectric ceramics are constituted by conventional PZT (zirconate/titanate) type piezoelectric elements included in a polymer matrix. For a detailed description of the technological principles involved in implementation, reference should be made to an article by Wallace Alden Smith published at pp. 755-766 of IEEE Ultrasonic Symposium, 1989.

The use of composite materials for making piezoelectric ceramics turns out to be advantageous since an acoustic impedance is obtained that is close to the acoustic impedance of biological tissues, and this translates into an increase in the sensitivity of the transducer and to a shorter impulse response. This enables such transducers to be used in medical echographic probes with the advantage of increasing the spatial resolution of such echographic probes.

This manufacturing technology also makes it possible to manufacture complex geometrical shapes, e.g. merely by thermoforming plane elements. Also, the use of a composite makes it possible to achieve transverse coupling that is very low such that the displacement of the sound-emitting surfaces is mainly unidirectional. This contributes to improving the electromechanical coupling and when the transducers are bar-shaped, it limits interactions between them, and this feature is particularly looked-for in echographic probes. It is also possible with composite materials to implement bar type transducers merely by integrating electrodes of the desired shape without cutting up piezoelectric elements.

As a result, composite materials are well-adapted to implementing medical echographic probes and to non-destructive monitoring where requirements are essentially centered on improving electromechanical coupling and spatial resolution for very low emission powers.

Heretofore, it has been believed that composite materials are not suitable for use in therapy (which requires high energy levels) because they suffer from a drop in performance at high excitation levels, as emphasized by Wallace Arden Smith in the above-specified article at p. 758. Mr. Smith emphasizes that at high energy levels transverse coupling increases and the piezoelectric element looses the unidirectional nature of its motion by acquiring transverse deformations that would make it impossible to use bar-type transducers, for example, even in medical echography. Further, firstly because of the increased transverse coupling and secondly because of the addition of polymer material, non-linear behavior of the transducer at high power is suggested.

GB-A-2 126 901 discloses a mosaic transducer formed of a plurality of transducer elements brased on a plate, and used for ultrasound hyperthermia. US-A-4 858 597 disclosed a mosaic transducer formed of large transducer elements embedded in a soft silicone rubber, used for lithotripsy.

Under such conditions, the person skilled in the art has had a prejudice against using composite piezoelectric elements for therapeutic treatment by means of ultrasound because high energy levels are required for that purpose.

In contrast, the present inventors have just discovered in a totally unexpected manner that composite piezoelectric elements are strong enough to enable them to emit sufficient energy to achieve therapeutic treatment, be that lithotrity (i.e. by means of a shock wave focused on a focus or target point), or for destroying or treating cells or tissue of a living being by the thermal effect that is obtained from focused ultrasound.

It is recalled here that the energy or power levels required for medical therapy are much greater than those needed for medical imaging. For example, for lithotrity, hyper thermia, or tissue removal, the (pulse) energy levels are more than 10,000 times greater than those needed by medical imaging. In terms of power per pulse, these same levels are from 10 times to 1,000 times greater. For example, the energy level that needs to be transmitted for medical imaging is typically less than 1 ten thousandth of a J per pulse. For therapeutic treatment, the energy that needs to be transmitted by the piezoelectric elements is significantly greater than 0.01 J per pulse. For lithotrity the energy level required generally lies in the range 0.01 J per pulse to 1 J per pulse. For medical hyperthermia and for thermal tissue removal, the energy required is generally in the order of several tens of Joules.

### SUMMARY OF THE INVENTION

Thus, in a first aspect, the present invention provides the use of at least one composite piezoelectric transducer in the manufacture of an ultrasonic therapy apparatus for applying destructive treatment, in a body zone, according to claim 1.

In a particular embodiment, the above-specified composite piezoelectric transducer is a single element of large size.

In another particular embodiment, the emitting surface of the above-specified transducer is split up into a plurality of emitting surfaces capable of being excited separately.

In yet another particular embodiment, a multiplicity of individual composite piezoelectric transducers are used which are assembled together to form a device comprising at least as many emitting surfaces as it has individual transducers. Such a device may be called a "multi-transducer" device.

In another particular embodiment, natural and/or electronic focusing means are provided for focusing the acoustic or ultrasonic waves emitted by the emitting surfaces of the above-mentioned transducers.

Advantageously, provision may be made for the above-mentioned composite piezoelectric transducers to emit ultra sound waves that are focused on a focal point or "target" point. This focusing may be achieved electronically or naturally by providing the transducer(s) or a multi-transducer device that is physically in the form of a naturally-focusing spherical cup. When electronic focusing is used, the transducers are fed electrically with predetermined phase shifts.

The unexpected effects of using composite piezoelectric ceramic transducers for therapeutic treatment are the following:
even at high energy, it is observed that composite piezoelectric materials conserve satisfactorily linear behavior; furthermore, given that the composite materials include polymer material, thermal dissipation is less than with conventional ceramic transducers, which would appear, a priory, to prevent them being used in high energy applications, it has now been discovered that it is possible to achieve thermal dissipation that is sufficient for enabling therapeutic treatment to be performed; and
by using composite technology, it is possible to manufacture multi-transducer devices having a filling factor of close to 100% whereas in conventional technology using a mosaic of ceramic transducers, it is typically not possible to do better than about 66%.

In another particular embodiment of the invention, a mono- or multi-transducer composite piezoelectric device is provided made in the form of a cup that fits substantially over the shape of the body of a patient and the apparatus is caused to emit an essentially spherical wave by phase synthesis introducing phase delays that enable an essentially spherical wave to be synthesized.

At present, a composite piezoelectric transducer which turns out to be particularly advantageous for implementing an acoustic or ultrasonic therapy apparatuses for therapy in particular of, tissues of a living being, is obtained from sticks of piezoelectric ceramic based on an alloy of zirconate and titanate, in particular an alloy of lead, zirconate and titanate (PZT) included in a compatible polymer matrix selected from the group comprising: an epoxy resin, a silicone and a polyurethane, and preferably an epoxy resin or a polyurethane.

The technology for making composite piezoelectric materials is well known to the person skilled in the art and has been described for the most part in the article by Wallace Arden Smith, published at pp. 755-766 in IEEE Ultrasonic Symposium, 1989.

For example, one of the commonly used techniques is the so-called "dice and fill technique" which consists in starting from a PZT (lead zirconate titanate) type piezoelectric ceramic and in cutting fine grooves in the thickness thereof to form a regular grating of ceramic sticks. The grating is then impregnated with the polymer component which is of the silicone type, the epoxy resin type, or the polyurethane type. Once the resin has polymerized, the material is adjusted to the desired thickness by cutting its base. A grating of independent sticks included in a polymer matrix is then obtained. The following step consists in depositing transducer feed electrodes on each face, e.g. by metal plating in a vacuum. By using masks, it is possible to deposit a single electrode for the entire transducer or a plurality of adjacent electrodes in order to subdivide the transducer assembly into a plurality of groups that can be excited separately. Thereafter, a final polymer layer is deposited which has two functions: electrical insulation and acoustic matching. It is important to ensure that the successive deposits adhere securely to one another. Finally, in a final step, the transducer is shaped to give it an arbitrary shape, e.g. a spherical shape. It should be observed that this operation is sometimes performed before the metal plating stage.

The volume occupied by the piezoelectric material relative to the total volume may lie in the range 8% to 70%. According to the present invention, it is advantageous for the volume proportion of the piezoelectric material relative to the total volume to lie in the range 20% to 40%.

The invention also provides an acoustic or ultrasonic therapy apparatus for applying destructive treatment, in a body zone, according to claim 13. The high level of ultrasonic or acoustic energy that the feed means are capable of sending to said transducer is not less than 0.01 J per pulse during a period of time that is sufficient for performing said therapy for treating tissues such as benign or malign tumors, or for treating bones, in particular at fractures or at zones to be treated such as zones of osteoporosis. The invention may also be used for treating varicose veins.

In a variant embodiment, the above-mentioned composite piezoelectric transducer may be disposed outside the body or else it may be mounted on or it may form an integral portion of an endo-cavity probe capable of being inserted into a cavity of the body of a living being.

In another particular variant embodiment, the above-mentioned composite piezoelectric transducer is used to perform medical hyperthermia or thermotherapy or to thermally remove tissue such as tumor tissue by volatilization thereof.

It is possible to provide a multi-transducer composite piezoelectric device for the combined purposes of therapy and of imaging. In a first case, a determined number of transducers enable echographic images to be formed (advantageously by associated electronic means permanently connected thereto) while the other transducers perform the therapeutic function. In a particularly advantageous embodiment, the transducers performing the imaging function may be disposed along one or more emitting lines so as to form one or more image planes that are preferably orthogonal. This disposition is particularly advantageous, in particular because it provides one or more image planes that pass permanently through the focal point when the above-mentioned emitting lines are disposed in a plane of symmetry of the multi-transducer apparatus, thus making it possible to obtain continuous imaging in real time of the therapeutic process.

In a second case, a determined number of the transducers or even all of them provide both an imaging function and a therapeutic function. These two functions may be obtained sequentially by appropriate sequencing control means that are well known to the person skilled in the art.

It should be observed that using composite piezoelectric transducers of the invention in a combined therapy and imaging application is particularly advantageous when associated with the method and device for locating and wave focusing as described in Document FR-89 01 628.

Composite piezoelectric transducers of the invention, in particular multi-transducer apparatuses for combined therapy and imaging use, may be applied to ultrasonic hyperthermia treatment of benign or malign tumors of the liver, of the kidney, the prostate, the breast, and also to treatment of varicose veins.

The composite piezoelectric transducer(s) of the invention may advantageously be integrated in an endo-cavitary probe, in particular a transrectal probe. In this context, the invention advantageously serves to treat cancer of the prostate.

Such an endo-cavitary probe preferably has the structure described in the Applicants' prior patent application FR-91 02 620 filed March 5, 1991, with number publication FR-A-2.673.542.

### BRIEF DESCRIPTION OF THE DRAWINGS

Other objects, characteristics, and advantages of the invention appear clearly in the light of the following explanatory description made with reference to the presently preferred embodiment of the invention which is given merely by way of example and which does not limit the scope of the invention in any way. In the drawings:
Figure 1 is a rear view of a spherical focusing cup implemented using a multi-transducer composite piezoelectric apparatus having 25 individual transducers numbered 1 to 25 (large digits) which are subdivided into five distinct groups numbered 1 to 5 (small digits);
   it should be observed that the first transducer numbered 1 (large digit) constitutes, on its own the group number 1 (small digit), with this cup-shaped device constituting the essential portion for emitting focused ultrasonic waves in combination with other essential members shown in Figure 2;
Figure 2 is a block diagram of an acoustic or ultrasonic therapeutic apparatuses of the present invention for applying therapy to tissue of a living being and constituting an application of the Figure 1 multi-transducer apparatus having composite type transducers;
Figure 3 is a graph showing the linearity obtained with the central composite piezoelectric transducer number 1 (large digit) constituting the first group in Figure 1; the maximum pressure obtained in bars is plotted up the Y-axis as a function of the applied voltage expressed in volts;
Figure 4 is a graph showing the aging of the number 1 composite piezoelectric transducer of Figure 1 as obtained at a given pressure expressed in bars and plotted up the Y-axis as a function of the number of shots multiplied by 1,000 plotted along the X-axis (curve plotted with asterisks at *-*), and secondly the impedance in ohms plotted up the righthand Y-axis as a function of the number of shots multiplied by 1,000 plotted along the X-axis (curve o-o);
Figure 5 is a diagram showing a second embodiment of an acoustic or ultrasonic therapy apparatus performing both imaging and therapy, and
Figure 6 is a block diagram of a third embodiment of an acoustic or ultrasonic therapeutic apparatus of the present invention for applying therapy to tissue of a living being, comprising a multitransducer device having composite type transducers according to an annular arrangement of the emitting surface.

### DETAILED DESCRIPTION

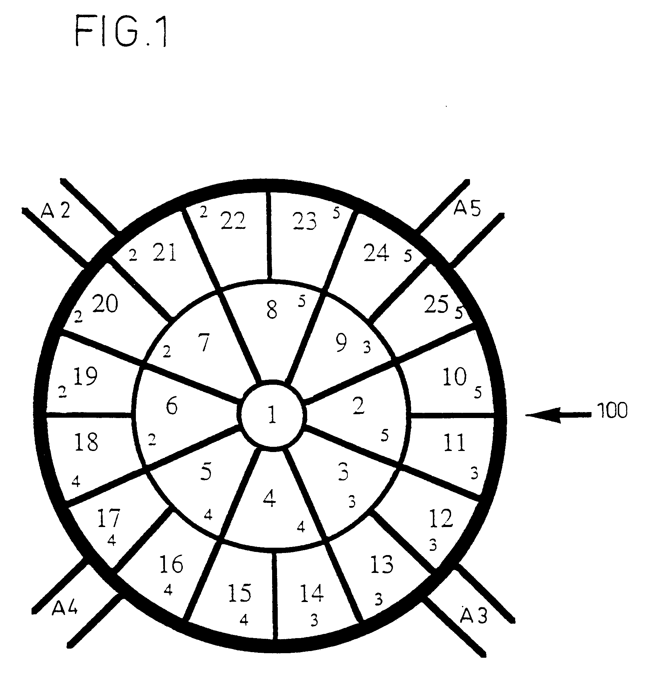

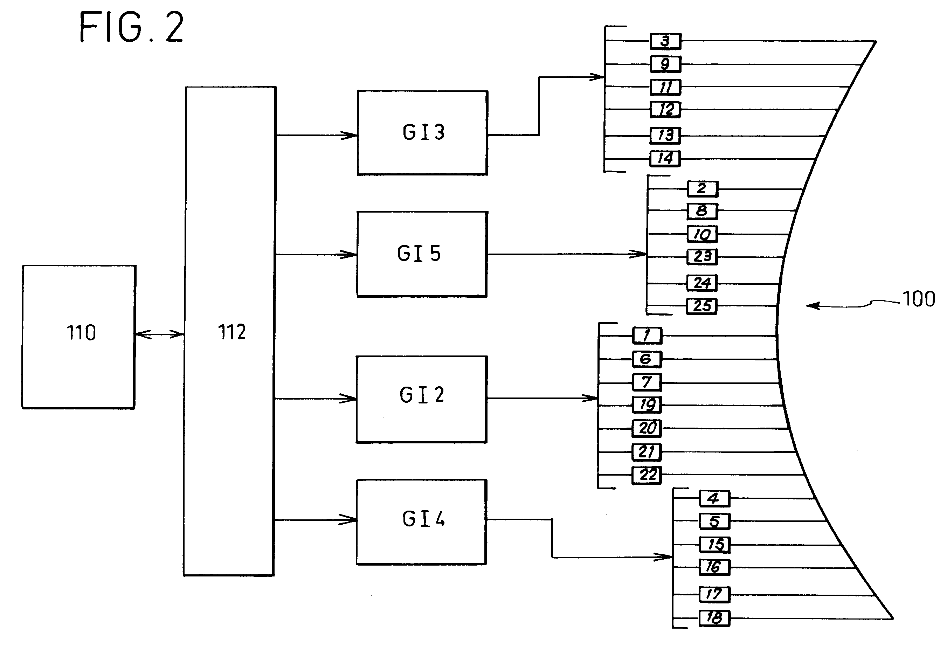

The composite multi-transducer device of Figure 1 is given overall reference numeral 100. The device 100 is made using the method described above. In this particular case, the electrodes are deposited using a mask that defines 25 electrodes that are adjacent and independent and of the shape shown in Figure 1, thereby forming 25 individual transducers that are capable of being fed separately. The assembly was then shaped to form, in the example, a cup having an opening of 100 mm and a radius of curvature of 160 mm. The piezoelectric material is of the PZT type and it constitutes about 25% of the total volume. The 25 transducers respectively numbered 1 to 25 (large digits) are subdivided into five subgroups numbered 1 to 5 (small digits) which are fed in groups and individually as described below with reference to Figure 2. The feeds to the groups 2, 3, 4, and 5 are deposited on the sides and are referenced A₂, A₃, A₄, and A₅ respectively.

The transducer 1 constitutes a group on its own, and for practical reasons it is combined with group No. 2 to be fed from feed A₂.

The technique for making the multi-transducer composite device is conventional and as described by Wallace Arden Smith at pp. 755-766 of IEEE Ultrasonic Symposium, 1989, and in particular with reference to Figure 2.

Accompanying Figure 2 shows a block diagram of the cup-shaped multi-transducer device 100 of Figure 1 together with its electronic feeds. It can be seen that each individual composite piezoelectric transducer of the device 100 is associated with a pulse transformer which is given the same reference numeral as the number of the individual transducer that it feeds.

There are therefore as many pulse transformers as there are transducers in the cup.

Thus, in this case, there are 25 pulse transformers which are grouped together to form four groups fed by four pulse generators. In this case, the four main groups 2, 3, 4, and 5 are shown together with their associated pulse generators referenced GI2, GI3, GI4, and GI5. These pulse generators are controlled by a central control unit 110, e.g. in the form of a computer and which contains, in the form of a file, all of the information necessary for individually controlling each of the 25 individual transducers. This is done by attributing control parameters to the transducers of the various electronic paths, and in particular phase delays ad firing management, e.g. with respect to the number of shots and to the firing rate. This data is sent by the central control unit 110 to the pulse generator cards which are referred to as "pulse generators" via an interface card 112, as shown in Figure 2.

Each pulse generator card can control six to seven transducers independently. It is therefore necessary in this case to provide at least four cards, given that the central transducer 1 which constitutes a group on its own is controlled by the pulse generator referenced GI2.

It will be understood that the pulses which are sent by the pulse generators are received by the pulse transformers for amplification and are transmitted to the transducers individually, thereby providing a high degree of flexibility in operation and making it possible to adjust the overall power of the multi-transducer composite piezoelectric device 100 at will.

The central individual piezoelectric element referenced 1 in Figure 1 has been used to perform tests on the linearity of the sensitivity of the transducer element in terms of positive pressure P+ as a function of the applied voltage.

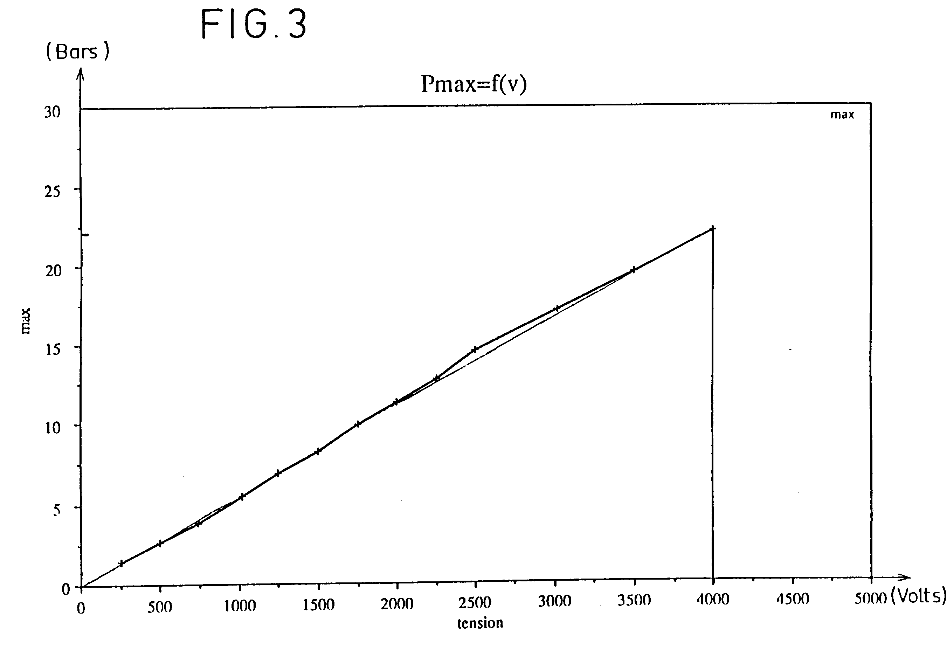

The results of these tests are given in the form of curves in Figure 3 where volts are plotted along the X-axis and pressure in bars is plotted up the Y-axis giving the maximum pressure obtained as a function of the applied voltage. The resulting curve has a slope of 5.6 mbars/volt, which is remarkable.

It is also surprising to observe that this curve is extremely linear regardless of the applied voltage and the pressure of the waves emitted to the focal point at the center of the cup, expressed in bars.

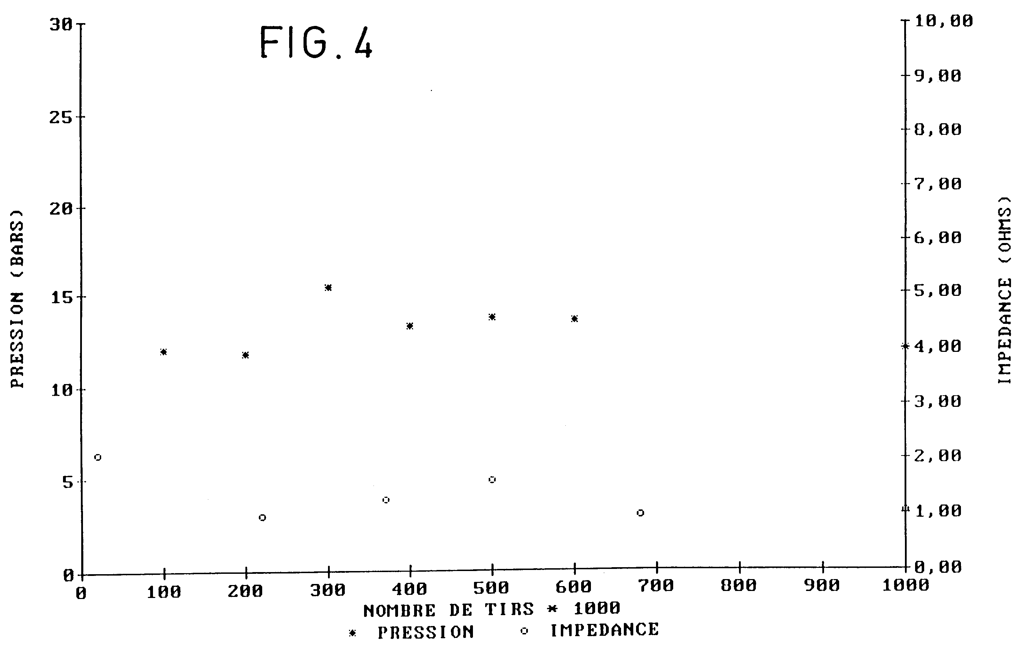

In addition, aging tests have also been performed using the composite piezoelectric transducer 1 of Figure 1, with these tests being shown in the form of a curve in Figure 4 where number of shots X 1,000 is plotted along the X-axis and where the lefthand Y-axis indicates pressure in bars. The curve showing pressure as a function of shots is plotted with asterisks *. The righthand Y-axis gives impedance in ohms and the corresponding curve is plotted using small circles o.

It can be seen that at an initial pressure of about 13 bars, this can be maintained essentially constant regardless of the number of shots, which is quite remarkable.

Similarly, the impedance of the composite piezoelectric material is kept essentially constant at around 1 ohm regardless of the number of shots, which is likewise remarkable and unexpected for the person skilled in the art.

The invention thus makes it possible to achieve all of the above-announced determining technical advantages.

In particular, the composite piezoelectric transducer of the invention, in particular when in the form of a multi-transducer device, can be used for making an acoustic or an ultrasonic therapy apparatus for therapy that may be destroying a concretion, destroying tissue such as a tumor, or treating bones.

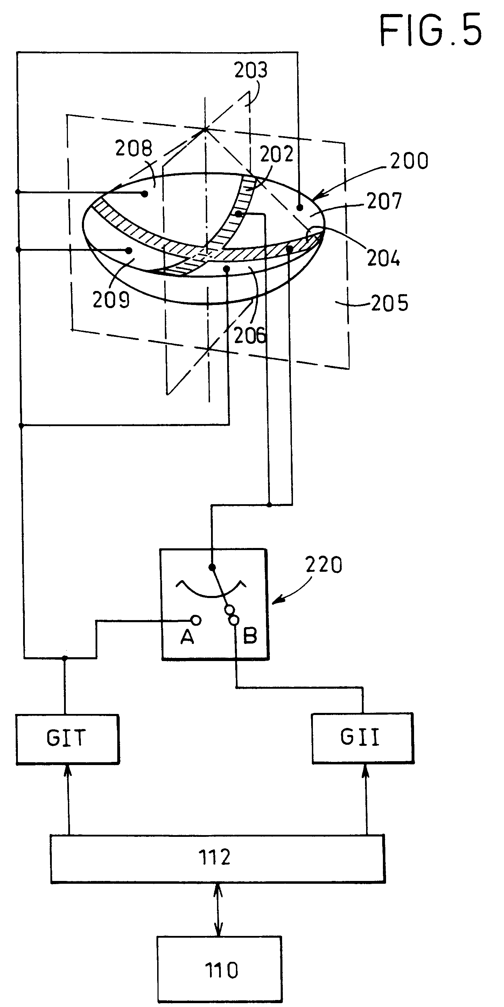

With reference to Figure 5, there can be seen a second embodiment of therapy apparatus of the present invention which has combined therapy and imaging functions, comprising a multi-transducer device of a type similar to that shown with reference to Figures 1 and 2, in the form of a cup, but in which a plurality of transducers associated with an imaging function are disposed, in this case in two perpendicular lines 202 and 204 occupying planes of symmetry of the cup 200, thereby obtaining image planes 203 and 205 that permanently contain the focal point F or center of the naturally-focusing spherically-shaped cup 200.

In addition, the other transducers of the cup 200 are distributed over four preferential sectors respectively referenced 206, 207, 208, and 209 and which perform a therapy function.

In this case also, there is a central control unit 110, e.g. a computer, controlling (via an interface 112) therapy pulse generators (GIT) or an imaging pulse generator (GII) respectively connected to the sectors of therapy transducers 206, 207, 208, and 209, or to the imaging transducers disposed on the lines 202 and 204 which are disposed orthogonally in this case. In a particularly preferred variant, a switch device 220 is included having a position A and a position B. When the switch is in its position A, the therapy pulse generator also causes the transducers in the imaging lines 202 and 204 to generate therapy pulses, i.e. when the switch is in position A, all of the transducers in the cup perform therapy, i.e. they operate at high power. In contrast, when the switch is in its position B, as shown in Figure 5, the therapy pulse generator controls the transducers in the therapy sectors 206, 207, 208, and 209 only, while the imaging pulse generator GII controls the transducers disposed in the emitting lines 202 and 204 for imaging purposes so as to construct an echographic image of the region around the focus F occupying two perpendicular planes each including the point F.

It will easily be understood that with the apparatus as shown in Figure 5, it is possible by appropriate programming of the interface 112 by the control unit 110 when the switch is in position A to make the pulse generators operate therapeutically to provide a sequence of therapy pulses per se controlling all of the transducers in the cup 200, or else under reduced power in an imaging mode to control all of the transducers in the cup 200 at an imaging power. The apparatus thus provides most appreciable versatility in use.

According to the aforesaid method for the manufacture of the composite piezoelectric transducers, according to the invention, it has also been manufactured a transducer under the shape of a single element spherical cup having a focal of 35 mm and a diameter of 5O mm, this diameter being optionally reduced in the transverse direction to 35 mm through a bilateral cutting for the purpose of reducing the transverse size, which is advantageous when it is wished to introduce this transducer in the form of a cup inside the body of a living being, into a corporeal orifice such as the rectum. The opening of this transducer having such a ratio between the diameter and the focal can only be obtained with a piezocomposite technology.

Similarly, it was manufactured a transducer under the shape of a single element spherical cup with a diameter of 28O mm and a focal of 19O mm. Here again, the opening as well as the dimensions of such a transducer can only be obtained with a piezocomposite technology.

The tests, which have been performed to determine the resistance to a working under power of the piezocomposite transducer according to the invention, have shown that the invention piezocomposite transducers can support powers up to 15 watts per cm² under a continuous working and that the damage threshold only appears from 3O watts per cm2.

These values are very high and are unexpectedly obtained for one skilled in the art as previously emphasized in the introductive portion of the description, on pages 1 to 3.

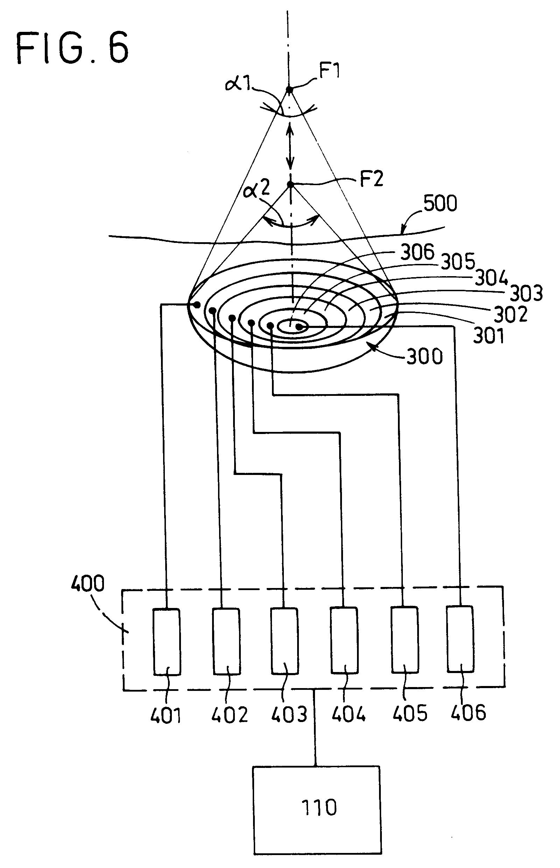

With reference to figure 6, it has been shown another embodiment of the therapy apparatus according to the invention for performing a therapy notably of concretions, tissues and bones of a living being, comprising similarly to figures 2 and 5, a piezocomposite transducer 3OO in the shape of a naturally focusing spherical cup focusing to the geometrical focal point F1. The emitting surface of the transducer 3OO is sub-divided into several emitting surfaces, here reaching the number of 6, respectively referenced 3O1, 3O2, 3O3, 3O4, 3O5, 3O6, having a concentric annular shape, which can be separately excited through the aid of individual exciting electronic circuits respectively referenced 4O1, 4O2, 4O3, 4O4, 4O5, 4O6, inside an electronic box 4OO itself linked and controlled by a central control unit 11O. It can thus be understood that this sub-division allows to constitute an annular network of emitting surface.

Each elementary emitting surface, such as 3O1, etc, is individually fed through its associated electronical circuit such as 4O1, etc, which enables to apply a delay on the feeding signals according to means well-known to those skilled in the art and already used for instance in the field of medical echography.

These delays applied to each emitting surface permit to vary the focal distance of the transducer, for instance up to focal point F2, from the geometrical focal point F1 determined by the spherical geometry of the transducer.

The set of the individual electronical circuits 4O1 to 4O6 constitutes focusing electronical means 4OO, which, according to a specific embodiment, can be used simultaneously to natural focusing means lying in the form of a spherical cup of the piezocomposite transducer 3OO. The focusing means are controlled by the central control unit 11O for example a computer, which has been previously described.

It can be understood that the use of piezocomposite materials allow for the first time to perform easily, very precisely, in a reproducible manner and at a less cost this type of transducer having an emitting surface under the shape of an annular network.

The invention therefore renders possible the manufacture of transducer having a variable opening from the opening angle alpha 1 to alpha 2, with a focal point having a variable position from F1 to F2.

These transducers with variable opening have a giant interest for the treatment by focused ultrasounds at high power. They permit to better protect against the thermal effects, the interfacing tissues located within the acoustic field of the transducer. Indeed, the opening of the transducer, determined by the ratio between diameter and a focal, is all the more important as the treatment is performed at a short distance. This increase in the opening allows on one hand to lower the focal spot of the transducer, and on the other hand to distribute the ultrasonic field onto a more important surface and to therefore lower the acoustical intensity onto the interfacing tissues represented by the general reference number 5OO.

According to a specific feature, it is possible to foresee feed-back control means of a type known to one skilled in the art enabling to feed-back control the opening of the transducer, thereby enabling to keep and to remain at not more than a predetermined acoustical intensity onto the interfacing tissue, such as interfacing tissue 5OO.

The piezocomposite transducer having an emitting surface sub-divided into an annular network can be integral with an endocavitary probe, not shown on figure 6, in particular an endorectal probe such as described in prior applicant French patent FR-9O.O4442.

This composite piezoelectric transducer 3OO can also be extracorporally located as it can be clearly understood by one skilled in the art.

The invention thus covers all means constituting technical equivalants to the means described and various combinations thereof.

It should be observed that all of the elements of the embodiments described with reference to Figures 1 to 6 form an integral portion of the invention and thus of the present description. The invention also covers any characteristic that appears to be novel over any of the prior art.

The composite piezoelectric transducer of the invention are used to perform medical hyperthermia or thermotherapy, or to damage tissue thermally, e.g. tumor tissue or varicose tissue, by causing a thermal effect selected from coagulation, necrosis, and volatilization thereof.

## Claims

1. Use of at least one composite piezoelectric transducer for the manufacture of a therapy apparatus for applying destructive treatment in a body zone, in particular to tissues of a living being, said destructive treatment comprising destructive treatment by thermal effect, hyperthermia, thermotherapy, or causing a thermal effect selected from coagulation, necrosis and volatilization, said apparatus having electrical feed means including pulse generators causing said transducer to provide a sufficient dosis of acoustic energy to perform said destructive treatment in said body zone.

2. The use of claim 1, wherein the said body zone comprises tumor tissues, begign or malign tumors of the liver, of the kidney, of the prostate, and of the breast, varicose tissues, prostate tissues.

3. The use of claim 1 or 2, wherein said dosis of acoustic energy is at least 0,01 J per pulse, and preferably in the order of several tens of Joules per pulse.

4. The use of one of claims 1 to 3, wherein the above-specified composite piezoelectric transducer is a single element of large size.

5. The use of one of claims 1 to 3, wherein the emitting surface of the above-specified transducer is split up into a plurality of emitting surfaces capable of being excited separately.

6. The use of one of claims 1 to 3, wherein a multiplicity of individual composite piezoelectric transducers are used which are assembled together to form a device comprising at least as many emitting surfaces as it has individual transducers.

7. The use of claim 5 or 6, wherein the above-mentioned composite piezoelectric transducers are designed to emit ultrasonic waves that are focused on a focal point or "target" point.

8. The use of anyone of claims 1 to 5, wherein natural and/or electronic focusing means are provided for focusing the acoustic waves emitted by the above mentioned transducer clement(s), and in particular by means of an embodiment in the form of a naturally-focusing spherical cup.

9. The use of one of claims 1 to 3, or anyone of claims 5 to 8, wherein a multi-transducer device is provided implemented in the form of a cup that fits substantially over the shape of the body of a patient, the device being caused to emit an essentially spherical wave by phase synthesis imparting phase delays between the transducers, thereby enabling an essentially spherical wave to be synthesized.

10. The use of anyone of claims 1 to 9, wherein the above-mentioned composite piezoelectric is obtained from a piezoelectric ceramic based on zirconate and titanate, in particular on lead, zirconate, and titanate, known as PZT, included in a matrix of a compatible polymer selected from the group comprising an epoxy resin, a silicone, and a polyurethane.

11. The use of claim 10, wherein the volume percentage of the PZT ceramic relative to the total volume is 8% to 70%, preferably 20 to 40 %.

12. The use of one of claims 1 to 3, or anyone of claims 5 to 11, wherein the emitting surface of the transducer is sub-divided into a plurality of annular emitting surfaces which can be separately excited.

13. Acoustic or ultrasonic therapy apparatus for applying destructive treatment in a body zone, in particular to tissues of a living being, said destructive treatment comprising medical hyperthermia or thermotherapy, or to damage tissue thermally such as tumor tissue, or varicose veins by coagulation, necrosis, or volatilization thereof, the apparatus comprising at least one piezoelectric transducer, and electrical feed means including pulse generators for causing said transducer to provide a sufficient dosis of acoustic energy to perform said destructive treatment in said body zone, said piezoelectric transducer being made at least in part of a composite piezoelectric transducer.

14. The apparatus of claim 13, wherein the above-mentioned feed means are capable of causing said transducer to emit a high level of energy of at least 0.01 J per pulse and preferably in the order of several tens of Joules per pulse, during a sufficient period of time to perform said destructive therapy, in particular on tissues such as benign or malign tumors, or varicose veins.

15. The apparatus of claim 13, wherein the said apparatus is an apparatus for applying extracorporal therapy in a body zone.

16. The apparatus according claim 13 or 14, wherein the above-mentioned composite piezoelectric is mounted or forms an integral portion of an endo-cavity probe capable of being inserted into a cavity of the body of a living being.

17. The apparatus of claim 13, wherein the above-mentioned composite piezoelectric transducer is made in the form of a device having multiple transducers.

18. The apparatus of claim 17, wherein the composite piezoelectric transducer has a variable opening in particular by being shaped as a multitransducer device having an emitting surface sub-divided into several annular emitting surfaces.

19. The apparatus of claim 17 or 18, wherein the multiple transducer device is for use both in therapy and in imaging, a determined number of transducers cooperate with auxiliary electronic means to form echographic images while the other transducers perform the therapeutic function independently.

20. The apparatus of claim 19, wherein a switch device enables the transducers to operate sequentially to form images by echography and to perform the therapy in said body zone.

21. The apparatus of claim 19 or 20, wherein the transducers that perform the imaging function are disposed on one or more emitting lines so as to form one or more image planes which are preferably orthogonal and which include the focal point.

22. The apparatus according to anyone of claims 13 to 21, including a control unit controlling the above mentioned transducers individually, or in groups.

23. The apparatus according to anyone of claims 13 to 22, wherein the composite piezoelectric transducer is obtained from a piezoelectric ceramic based on zirconate and titanate, in particular on lead, zirconate, and titanate, known as PZT, included in a matrix of a compatible polymer selected from the group comprising an epoxy resin, a silicone, and a polyurethane.

24. The apparatus according to claim 23, wherein the volume percentage of the PZT ceramic relative to the volume of the total volume is 8% to 70%, preferably 20 to 40 %.

## Patentansprüche

1. Verwendung mindestens eines piezoelektrischen Kompositwandlers für die Herstellung einer Therapievorrichtung zur Anwendung destruktiver Behandlung in einem Körperbereich, insbesondere von Gewebe eines Lebewesens, wobei die destruktive Behandlung eine destruktive Behandlung durch thermische Effekte, Hyperthermie, Thermotherapie umfasst, oder einen thermischen Effekt hervorruft, der ausgewählt ist aus Koagulation, Nekrose, und Verdampfung, wobei die Vorrichtung Mittel für die elektrische Speisung einschließlich Pulsgeneratoren aufweist, welche dazu führen, daß der Wandler eine für die Ausführung der destruktiven Behandlung in dem Körperbereich ausreichende Dosis akustischer Energie liefert.

2. Die Verwendung nach Anspruch 1, wobei der Körperbereich Geschwulstgewebe, gutartige oder bösartige Geschwulste der Leber, der Niere, der Prostata und der Brust, variköse Gewebe und Prostatagewebe umfasst.

3. Die Verwendung nach Anspruch 1 oder 2, wobei die Dosis akustischer Energie mindestens 0,01 J pro Puls beträgt, und vorzugsweise in der Größenordnung von einigen Zehnern Joule pro Puls liegt.

4. Die Verwendung nach einem der Ansprüche 1 bis 3, wobei der oben angegebene piezoelektrische Kompositwandler ein einzelnes Element großer Abmessung ist.

5. Die Verwendung nach einen, der Ansprüche 1 bis 3, wobei die strahlende Fläche des oben angegebenen Kompositwandlers aufgeteilt ist in eine Mehrzahl von strahlenden Flächen, welche getrennt angeregt werden können.

6. Die Verwendung nach einem der Ansprüche 1 bis 3, wobei eine Mehrzahl einzelner piezoelektrischer Kompositwandler verwendet werden, welche zusammengefügt werden, um eine Vorrichtung zu bilden, die mindestens ebensoviele strahlende Flächen umfasst, wie sie einzelne Kompositwandler aufweist.

7. Die Verwendung nach einem der Ansprüche 5 oder 6, wobei die oben angegebene Kompositwandler ausgelegt sind, um Ultraschallwellen auszusenden, die sich in einem Brennpunkt oder "Target"punkt fokussieren.

8. Die Verwendung nach mindestens einem der Ansprüche 1 bis 5, wobei natürliche und/oder elektronische Mittel vorgesehen sind, um die von den, oben angegebenen Wandlerelement(en) ausgesandten akustischen Wellen zu fokussieren, und insbesondere mittels eines Ausführungsbeispiels in Form einer naturgemäß fokussierenden sphärischen Schale.

9. Die Verwendung nach einem der Anspräche 1 bis 3, oder nach mindestens einem der Anspräche 5 bis 8, wobei eine Multiwandler-Vorrichtung vorgesehen sist, die in Form einer Schale ausgeführt ist, welche im wesentlichen der Körperform eines Patienten paßgenau ist, wobei bei der Vorrichtung durch Phasensynthese hervorgerufene Phasenverzögerungen zwischen den Wandlers die Aussendung einer im wesentlichen sphärischen Welle verursacht wird, wodurch die Erzeugung einer im wesentlichen sphärischen Welle ermöglicht wird.

10. Die Verwendung nach mindestens einem der Anspräche 1 bis 9, wobei der oben angegebene piezoelektrische Kompositwandler erhalten wird aus einer piezoelektrischen Keramik auf der Basis von Zirkonat und Titanat. insbesondere von Blei, Zirkonat, und Titanat, bekannt unter der Bezeichnung PZT, das in einer Matrix aus einen, kompatiblen Polymer enthalten ist, welches ausgewählt ist aus der Gruppe bestehend aus einen, Epoxydharz, einem Silikon, und einem Polyurethan.

11. Die Verwendung nach Anspruch 10, wobei der Volumenanteil an PZT-Keramik bezüglich des Gesamtvolumens 8 bis 70% beträgt, vorzugsweise 20% bis 40%.

12. Die Verwendung nach einem der Ansprüche 1 bis 3, oder nach mindestens einen, der Ansprüche 5 bis 11, wobei die strahlende Fläche des Wandlers unterteilt ist in eine Mehrzahl von ringförmigen strahlenden Flächen, die getrennt angeregt werden können.

13. Akustische oder Ultraschalltherapievorrichtung für die Anwendung einer destruktiven Behandlung in einem Körperbereich, insbesondere von Gewebe eines Lebewesens, wobei die destruktive Behandlung medizinische Hypothermie oder Thermotherapie umfasst, oder für die thermische Schädigung von Geweben wie Geschwulstgewebe, oder Krampfadern durch deren Koagulation, Nekrose, oder Verdampfung, wobei die Vorrichtung mindestens einen piezoelektrischen Wandler, und Mittel für die elektrische Speisung einschließlich Pulsgeneratoren umfasst, welche dazu führen, daß der Wandler eine für die Ausführung der destruktiven Behandlung des Körperbereichs ausreichende Dosis akustischer Energie liefert, wobei der piezoelektrische Wandler zumindest teilweise aus einem piezoelektrischen Kompositwandler besteht.

14. Die Vorrichtung nach Anspruch 13, wobei die oben angegebenen Speisungsmittel in der Lage sind, bei dem Wandler das Ausstrahlen hoher Energie von mindestens 0,01 J pro Puls und vorzugsweise in der Größenordnung von einigen Zehnern Joule pro Puls, für einer Zeitdauer hervorzurufen, die für die Ausführung einer destruktiven Therapie, insbesondere von gutartigen oder bösartigen Geschwulsten oder Krampfadern ausreichend ist.

15. Die Vorrichtung nach Anspruch 13, wobei die Vorrichtung eine Vorrichtung für das Anwenden außerkörperlicher Therapie an einem Körperbereich ist.

16. Die Vorrichtung nach Anspruch 13 oder 14, wobei das oben angegebene Kompositpiezoelektrikum an einer Endohohlraumsonde angebracht sind oder mit ihr einstückig ist, wobei die Sonde in eine Körperhöhlung eines Lebewesens eingeführt werden kann.

17. Die Vorrichtung nach Anspruch 13, wobei der oben angegebene piezoelektrische Kompositwandler in Form einer Vorrichtung mit mehreren Wandlern ausgebildet ist.

18. Die Vorrichtung nach Anspruch 17, wobei der piezoelektrische Kompaktwandler eine variable Öffnung aufweist, insbesondere indem er als Multiwandler-Vorrichtung mit einer in mehrere ringförmige strahlende Flächen aufgeteilten strahlenden Fläche ausgebildet ist.

19. Die Vorrichtung nach Anspruch 17 oder 18, wobei die multiple Wandlervorrichtung für die Verwendung sowohl zu Therapiezwecken als auch zu Abbildungszwecken vorgesehen ist, wobei eine bestimmte Anzahl von Wandlern mit zusätzlichen elektronischen Mitteln zusammenwirkt, um Ultraschallabbildungen zu bilden während die anderen Wandler die therapeutische Funktion unabhängig ausführen.

20. Die Vorrichtung nach Anspruch 19, wobei eine Schaltervorrichtung es ermöglicht, daß die Wandler nacheinander durch Ultraschall Abbildungen zu bilden und eine Therapie in dem Körperbereich durchzuführen.

21. Die Vorrichtung nach Anspruch 19 oder 20, wobei die Wandler, welche Abbildungsfunktionen durchführen, auf einer oder mehr Strahlungslinien angebracht sind, unter Bildung einer oder mehrerer Bildebenen, welche vorzugsweise orthogonal sind und den Brennpunkt einschließen.

22. Die Vorrichtung nach mindestens einem der Ansprüche 13 bis 21, umfassend eine Steuereinheit, die die oben angegebenen Wandler einzeln oder in Gruppen steuert.

23. Die Vorrichtung nach mindestens einem der Ansprüche 13 bis 22, wobei der piezoelektrische Kompositwandler erhalten wird aus einer piezoelektrischen Keramik auf der Basis von Zirkonat und Titanat, insbesondere von Blei, Zirkonat, und Titanat, bekannt unter der Bezeichnung PZT, das in einer Matrix aus einem kompatiblen Polymer enthalten ist, welche ausgewählt ist aus der Gruppe bestehend aus einem Epoxydharz, einem Silikon, und einem Polurethan.

24. Die Vorrichtung nach Anspruch 23, wobei der Volumenanteil an PZT-Keramik bezüglich des Gesamtvolumens 8 bis 70% beträgt, vorzugsweise 20% bis 40%.

## Revendications

1. Utilisation d'au moins un transducteur piézo-électrique composite pour la fabrication d'un appareil de thérapie pour appliquer un traitement destructif à une zone corporelle, en particulier à des tissus d'un être vivant, ledit traitement destructif comprenant le traitement destructif par effet thermique, par hyperthermie, par thermothérapie, ou la mise en oeuvre d'un effet thermique choisi parmi la coagulation, la nécrose et la volatilisation, ledit appareil comportant des moyens d'alimentation électriques comprenant des générateurs d'impulsion, obligeant ledit transducteur à fournir une dose suffisante d'énergie acoustique pour réaliser ledit traitement destructif dans ladite zone corporelle.

2. L'utilisation selon la revendication 1, dans laquelle ladite zone corporelle comporte des tissus tumoraux, des tumeurs bénignes ou malignes du foie, du rein, de la prostate ainsi que sein, des tissus variqueux, des tissus prostatiques.

3. L'utilisation selon la revendication 1 ou 2, dans laquelle ladite dose d'énergie acoustique est d'au moins 0,01 J par impulsion, et préférablement de l'ordre de plusieurs dizaines de Joules par impulsion.

4. L'utilisation selon l'une des revendications 1 à 3, dans laquelle le transducteur piézo-électrique composite spécifié ci-dessus est un élément unique de grande taille.

5. L'utilisation selon l'une des revendications 1 à 3, dans laquelle la surface émettrice du transducteur spécifié ci-dessus est éclatée en une pluralité de surfaces émettrices susceptibles d'être excitées séparément.

6. L'utilisation selon l'une des revendications 1 à 3, dans laquelle on utilise une multiplicité de transducteur piézo-électriques composites individuels qui sont assemblés ensemble pour former un dispositif comprenant au moins autant de surfaces émettrices qu'il existe de transducteurs individuels.

7. L'utilisation selon la revendication 5 ou 6, dans laquelle les transducteurs piézo-électriques composites mentionnés ci-dessus sont réalisés pour émettre des ondes ultrasoniques qui sont localisées sur un point focal ou point "cible".

8. L'utilisation selon l'une quelconque des revendications 1 à 5, dans laquelle des moyens de focalisation naturels et/ou électroniques sont prévus pour focaliser les ondes acoustiques émises par le ou les élément(s) transducteur(s) mentionné(s) ci-dessus, et en particulier au moyen d'un mode de réalisation sous la forme d'une coupole sphérique naturellement focalisante.

9. L'utilisation selon l'une des revendications 1 à 3, ou l'une quelconque des revendications 5 à 8, dans laquelle il est prévu un dispositif multitransducteurs réalisé sous la forme d'une coupole épousant sensiblement la forme du corps d'un patient, dispositif auquel on fait émettre une onde sensiblement sphérique par un dispositif de synthèse de phase introduisant des retards de phase entre les transducteurs, de manière à permettre de synthétiser une onde sensiblement sphérique.

10. L'utilisation selon l'une quelconque des revendications 1 à 9, dans laquelle le transducteur piézo-électrique composite précité est obtenu à partir d'une céramique piézo-électrique à base de zirconate et de titanate, en particulier de plomb, de zirconate et de titanate, dite PZT, incluse dans une matrice en un polymère compatible choisi parmi le groupe comprenant une résine époxyde, un silicone, et un polyuréthanne.

11. L'utilisation selon la revendication 10, dans laquelle le pourcentage en volume de la céramique PZT par rapport au volume total est de 8 à 70 %, préférablement de 20 à 40 %.

12. L'utilisation selon l'une des revendications 1 à 3, ou l'une quelconque des revendications 5 à 11, dans laquelle la surface émettrice du transducteur est subdivisée en une pluralité de surfaces émettrices annulaires qui peuvent être excitées séparément.

13. Appareil de thérapie acoustique ou ultrasonique pour appliquer un traitement destructif à une zone corporelle, en particulier à des tissus d'un être vivant, ledit traitement destructif comprenant l'hyperthermie ou la thermothérapie, ou pour réaliser une altération thermique de tissus tels que des tissus tumoraux, ou des veines variqueuses par coagulation, par nécrose ou par volatilisation de ces tissus, l'appareil comprenant au moins un transducteur piézo-électrique et des moyens d'alimentation électrique comprenant des générateurs d'impulsion, pour obliger ledit transducteur à fournir une dose suffisante d'énergie acoustique pour réaliser ledit traitement destructif dans ladite zone corporelle, ledit transducteur piézo-électrique était constitué au moins en partie d'un transducteur piézo-électrique composite.

14. L'appareil selon la revendication 13, dans lequel les moyens d'alimentation précités sont capables de faire émettre audit transducteur un niveau d'énergie élevé d'au moins 0, 01 J par impulsion, et de préférence de l'ordre de plusieurs dizaines de Joules par impulsion, pendant une période de temps suffisante pour réaliser ladite thérapie destructive, en particulier sur des tissus tels que des tumeurs bénignes ou malignes, ou des veines variqueuses.

15. L'appareil selon la revendication 13, dans lequel ledit appareil est un appareil pour appliquer une thérapie extra corporelle dans une zone corporelle.

16. L'appareil selon la revendication 13 ou 14, dans lequel le transducteur piézo-électrique composite précité est monté sur ou fait partie intégrante d'une sonde endocavitaire capable d'être introduite dans une cavité du corps d'un être vivant.

17. L'appareil selon la revendication 13, dans lequel le transducteur piézo-électrique composite précité est réalisé sous la forme d'un dispositif présentant des transducteurs multiples.

18. L'appareil selon la revendication 17, dans lequel le transducteur piézo-électrique composite présente une ouverture variable, en particulier du fait qu'il est conformé comme un dispositif multitransducteur présentant une surface émettrice subdivisée en plusieurs surfaces émettrices annulaires.

19. L'appareil selon la revendication 17 ou 18, dans lequel le dispositif transducteur multiple est destiné à être utilisé à la fois en thérapie et en imagerie, et dans lequel un nombre déterminé de transducteurs coopèrent avec des moyens électroniques auxiliaires pour former des images échographiques tandis que les autres transducteurs réalisent indépendamment la fonction thérapeutique.

20. L'appareil selon la revendication 19, dans lequel un dispositif interrupteur permet aux transducteurs de fonctionner séquentiellement pour former des images par échographie et pour réaliser la thérapie dans ladite zone corporelle.

21. L'appareil selon la revendication 19 ou 20, dans lequel les transducteurs qui réalisent la fonction imagerie sont disposés sur une ou plusieurs lignes émettrices, de manière à former un ou plusieurs plans d'image qui sont de préférence orthogonaux et qui comportent le point focal.

22. L'appareil selon l'une quelconque des revendications 13 à 21, comprenant une unité de contrôle contrôlant les transducteurs précités, individuellement ou en groupes.

23. L'appareil selon l'une quelconque des revendications 13 à 22, dans lequel le transducteur piézo-électrique composite est obtenu à partir d'une céramique piézo-électrique à base de zirconate et de titanate, en particulier de plomb, de zirconate et de titanate, dite PZT, incluse dans une matrice en un polymère compatible choisi parmi le groupe comprenant une résine époxyde, un silicone ou un polyuréthanne.

24. L'appareil selon la revendication 23, dans lequel le pourcentage en volume de la céramique PZT par rapport au volume total est de 8 à 70 %, préférablement de 20 à 40 %.
